# EUROPEAN PATENT APPLICATION

(11) **EP 4 759 233 A2**
(43) Date of publication of application: **17.06.2026**
(21) Application number: 25218658.0
(22) Date of filing: 26.11.2025
(51) Int. Cl.: A61B 5/252, A61B 5/28, A61B 5/00

(54) **SYSTEMS AND METHOD FOR A SUCTION ELECTRODE ASSEMBLY**

(30) Priority: 16.12.2024 US 202418983151
(71) Applicant: GE Precision Healthcare LLC, Waukesha, WI 53188 (US)
(72) Inventor: AROCKIARAJ, Sudhakar, 560066 Bengaluru (IN); VARGHESE, Oswin, 560066 Bengaluru (IN)
(74) Representative: Kilburn & Strode LLP

(57) **Abstract**

Various methods and systems are provided for a suction electrode assembly (200), comprising, an electrode housing (204) that surrounds and positions an electrode plate (302), the electrode housing being coupled to a vacuum system (112), a suction dome housing (202) configured to position the electrode housing and comprises a top portion (202a), a bottom portion (202b), and a conical middle portion (202c) with an axially varying wall thickness that couples the top portion and the bottom portion, and wherein the top portion is configured to withstand a force that depresses the top portion, the conical middle portion is configured to concentrically collapse in response to the top portion being depressed, and the bottom portion is configured to contact a surface.

## Description

### FIELD

Embodiments of the subject matter disclosed herein relate to generating electrocardiograms (ECGs), and more specifically, to ensuring electrodes for calculating ECGs are in contact with a skin of a patient.

### BACKGROUND

Heart disease has become the most common disease that affects humans worldwide. Each year millions of people die from heart attacks and an equal number undergo coronary artery bypass surgery or balloon angioplasty for advanced heart disease. Early detection and timely treatment may reduce the probability and/or severity of such events. Early detection may improve the quality of life and slow the progression of heart failure.

Non-invasive approaches to collect electrocardiograms (ECGs) may be used to assess a patient's heart condition, which may enable early detection of cardiac irregularities. An ECG may utilize surface electrodes, e.g., electrodes in contact with the patient's skin to monitor cardiac activity. The non-invasive methods may generate electrocardiograms to provide information on the normal and/or pathological physiology of the heart and may be used to diagnose one or more cardiac conditions, such as arrhythmias, cardiac infarctions, cardiac hypertrophy, etc.

### BRIEF DESCRIPTION

In one example, a system includes a suction electrode assembly comprising an electrode housing that surrounds and positions an electrode plate, the electrode housing being coupled to a vacuum system, a suction dome housing configured to position the electrode housing and comprises a top portion, a bottom portion, and a conical middle portion with an axially varying wall thickness that couples the top portion and the bottom portion, and wherein the top portion is configured to withstand a force that depresses the top portion, the conical middle portion is configured to concentrically collapse in response to the top portion being depressed, and the bottom portion is configured to contact a surface. In this way, the suction electrode assembly has sufficient holding force to ensure an electrode plate of the suction electrode assembly is in contact with a skin of a patient and electrical potential on the skin of the patient may be measured to generate electrocardiograms.

It should be understood that the brief description above is provided to introduce in simplified form a selection of concepts that are further described in the detailed description. It is not meant to identify key or essential features of the claimed subject matter, the scope of which is defined uniquely by the claims that follow the detailed description. Furthermore, the claimed subject matter is not limited to implementations that solve any disadvantages noted above or in any part of this disclosure.

### BRIEF DESCRIPTION OF THE DRAWINGS

The present disclosure will be better understood from reading the following description of non-limiting embodiments, with reference to the attached drawings, wherein below:
FIG. 1 shows a block diagram of an exemplary embodiment of an ECG system.
FIG. 2 shows an example of a suction electrode assembly in a non-actuated state.
FIG. 3 shows a first example of a first cross section of the suction electrode assembly of FIG. 2.
FIG. 4 shows a second example of the first cross section of the suction electrode assembly of FIG. 2.
FIG. 5 shows a second cross section of the suction electrode assembly of FIG. 2.
FIG. 6 shows an example of the suction electrode assembly in an actuated state.
FIG. 7 shows a first cross section of the suction electrode assembly of FIG. 6.
FIG. 8 shows a second cross section of the suction electrode assembly of FIG. 6.
FIG. 9 shows a flow chart for an example of a method for collecting ECG data and generating vectorcardiograms based on the ECG data from a suction electrode assembly, according to embodiments described herein.
FIG. 10 shows a first timing diagram for achieving an actuated state of a suction electrode assembly.
FIG. 11 shows a second timing diagram for achieving a non-actuated state of a suction electrode assembly.

### DETAILED DESCRIPTION

The following description relates to systems and methods for a suction electrode assembly wherein a suction dome housing encloses an electrode assembly. Existing suction dome housings do not have sufficient holding force demanded for attaching to skin, such as skin covered with hair, due to the stiffness of the aforementioned suction dome housings. Consequently, lateral forces transferred to the suction dome housing due to movement of the patient or movement of the lead wires when the lead wires are pulled may result in detachment of the suction dome housing, and thus, the suction electrode assembly from the skin of the patient. As such, muscle tremors (e.g., due to movement of the patient) and other movement of various components of the ECG system may introduce noise in the ECG data, which may render the ECG data as non-diagnostic.

Further, existing suction dome housings are configured to operate with vacuum systems that operate with constant flow rates and with non-continuous or intermittent vacuum systems that operate with variable flow rates. However, the existing suction dome housing does not consistently provide a sufficient holding force to maintain contact between an electrode plate of an electrode assembly and the skin of the patient when coupled to a non-continuous/intermittent vacuum system. For example, the suction dome housing may not provide sufficient holding force when the suction dome housing is coupled to the non-continuous/intermittent vacuum system and placed on soft muscle of the patient or wet skin of the patient (e.g., skin coated in sweat).

The inventors hereby recognize the disadvantages discussed above and hereby attempt to address these disadvantages with a suction electrode assembly and a method for operating the suction electrode assembly to obtain ECG data. The suction electrode assembly comprises a suction dome housing and an electrode housing that encloses an electrode plate wherein the suction electrode housing and the electrode assembly are arranged with an interference fit to ensure that an upper region of the suction electrode assembly is rigid. Moreover, the various features of the suction electrode housing, including wall thickness, height, diameter size, lip radius, surface finish, hardness, etc.) are configured to reduce muscle tremor, reduce air leaks, generate a trigger signal for different operating conditions, and increase comfort of a patient during operation of the suction electrode assembly.

In particular, the suction electrode housing includes a top portion, a bottom portion, and a conical middle portion that is spaced between the top portion and the bottom portion. The conical middle portion is configured with an axially variable wall thickness that is sufficiently flexible and enables the conical middle portion to concentrically collapse to achieve an actuated state that enables contact between the electrode plate of the suction electrode assembly and the skin of the patient.

The top portion and the bottom portion are configured to ensure that a shape of the top portion and a shape of the bottom portion are maintained when force is applied to the suction electrode assembly. Further, the bottom portion is sufficiently flexible and configured with a lip radius of a pre-determined size, or rather, the lip radius is large enough to reduce skin irritation of the patient when the suction electrode assembly is in the actuated state.

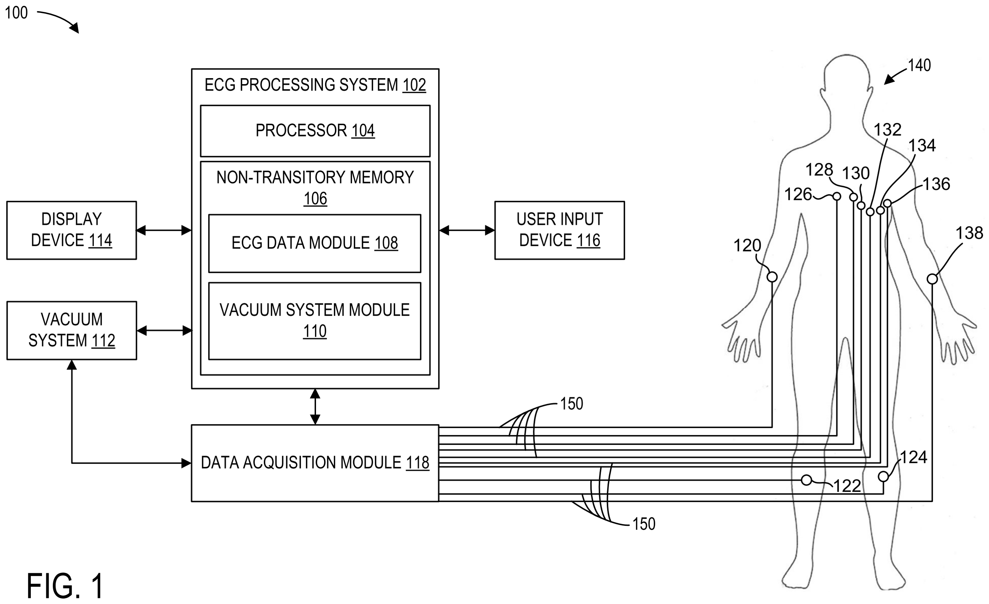

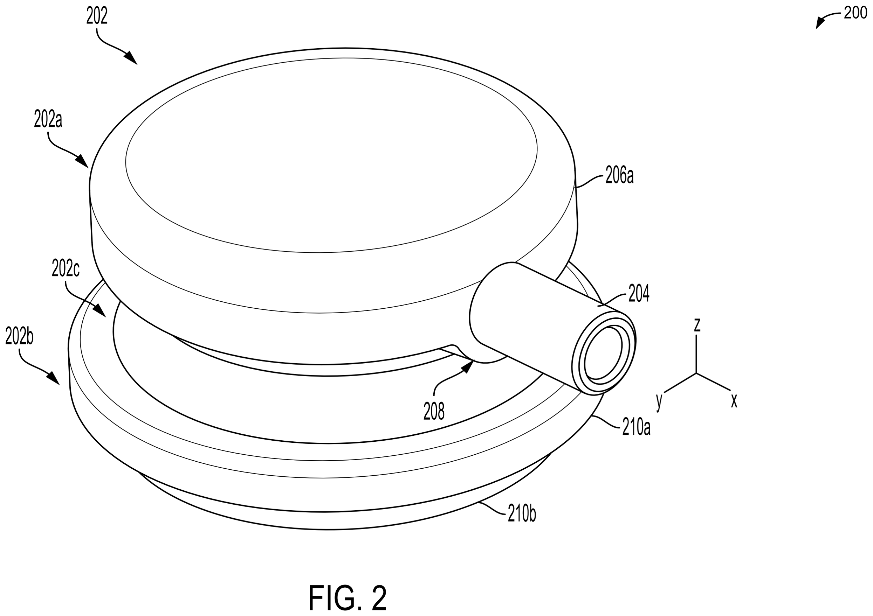

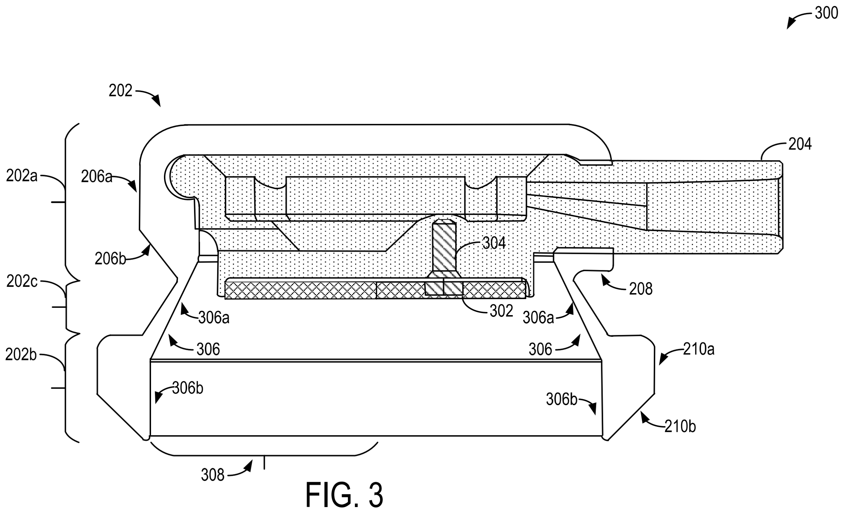

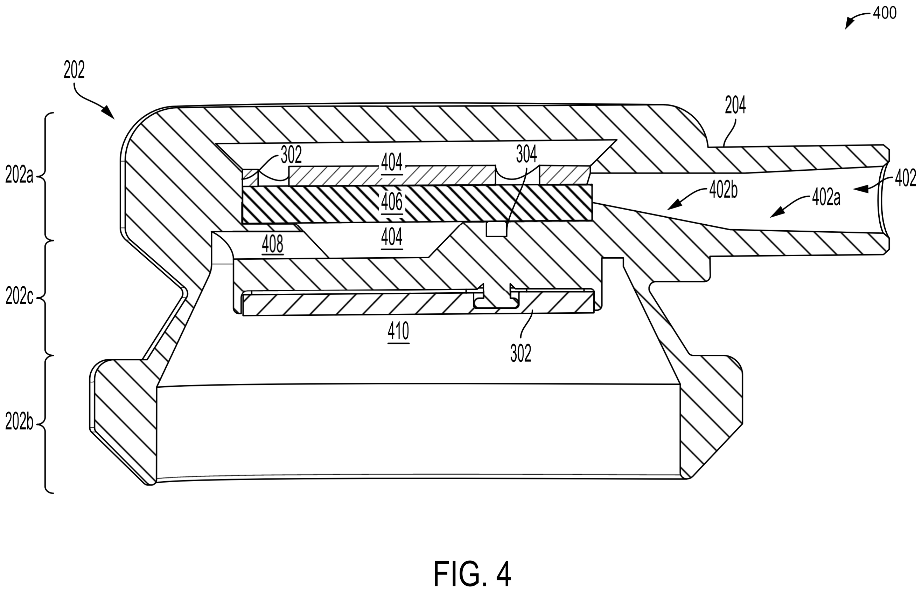

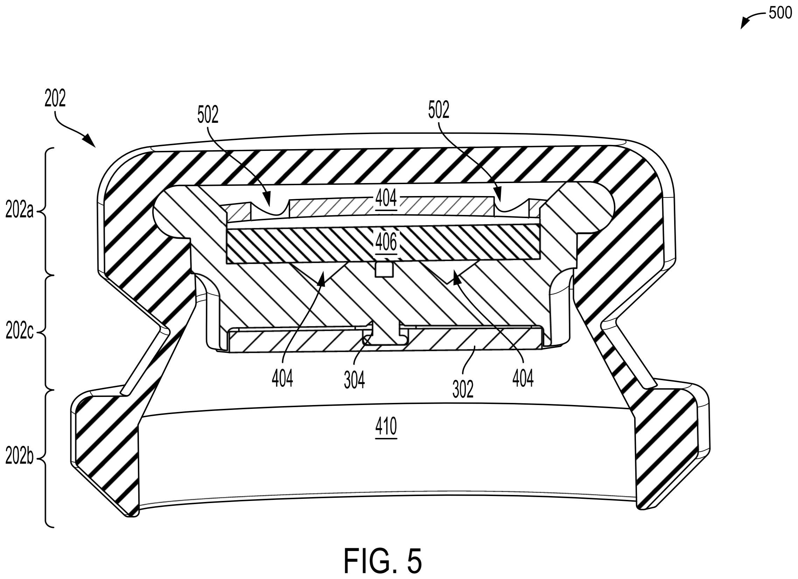

An example ECG system used to collect ECG data is shown in FIG. 1. The ECG system may be a surface ECG, where electrodes are placed on a patient's skin. FIG. 2 shows an example of a suction electrode assembly in a non-actuated state. A first example of a first cross section of the suction electrode assembly depicted in FIG. 2 is illustrated in FIG. 3. A second example of the first cross section of the suction electrode assembly depicted in FIG. 2. is illustrated in FIG. 4. FIG. 5 illustrates a second cross section of the suction electrode assembly depicted in FIG. 2.

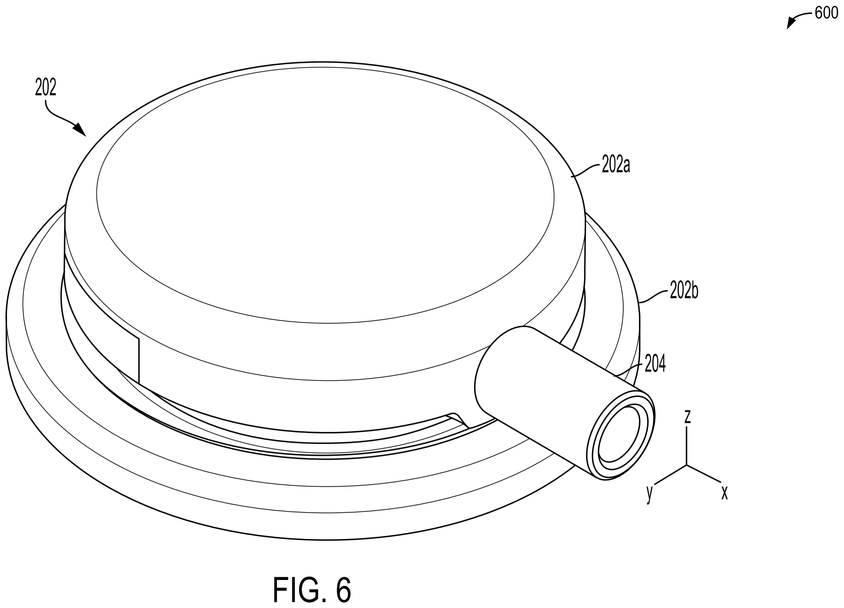

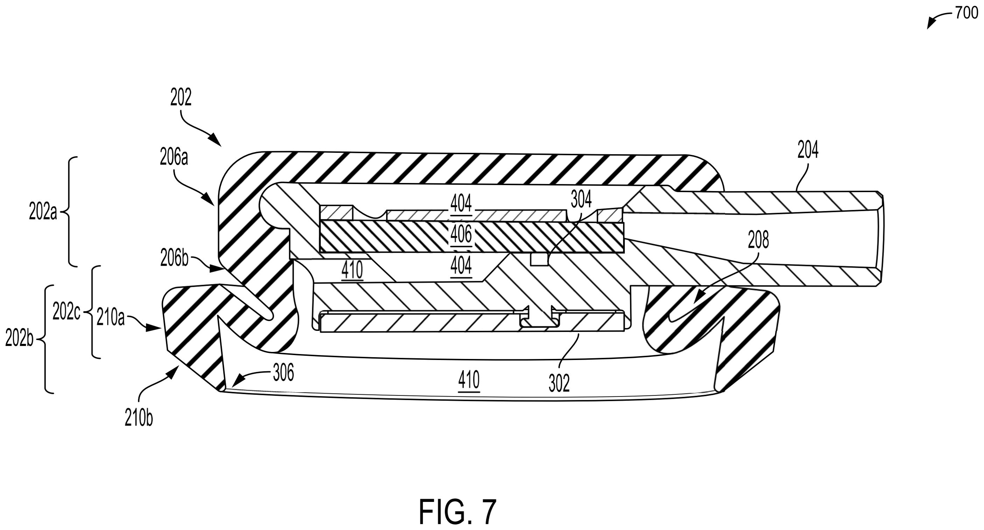

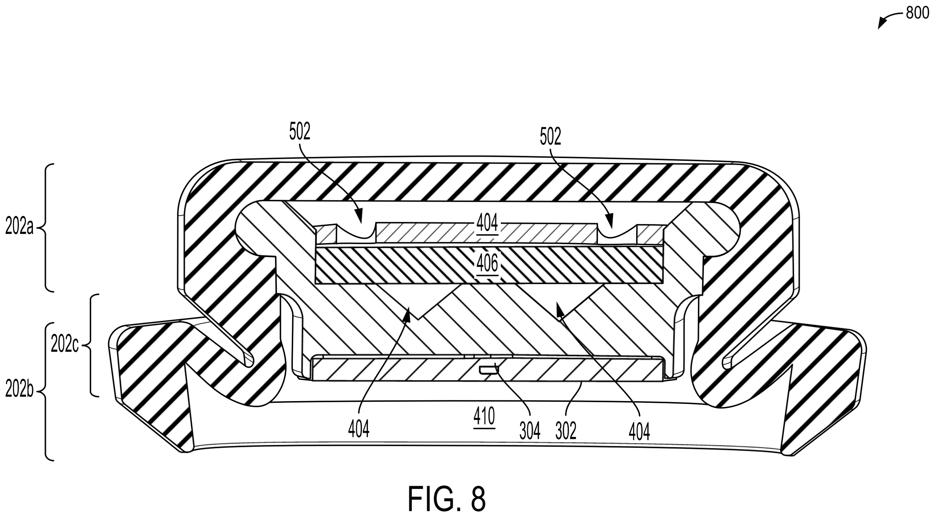

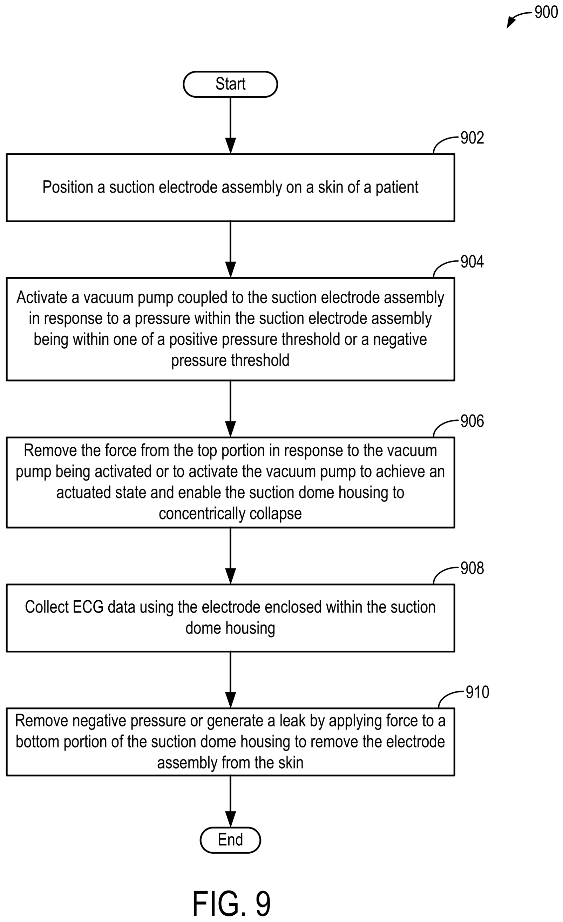

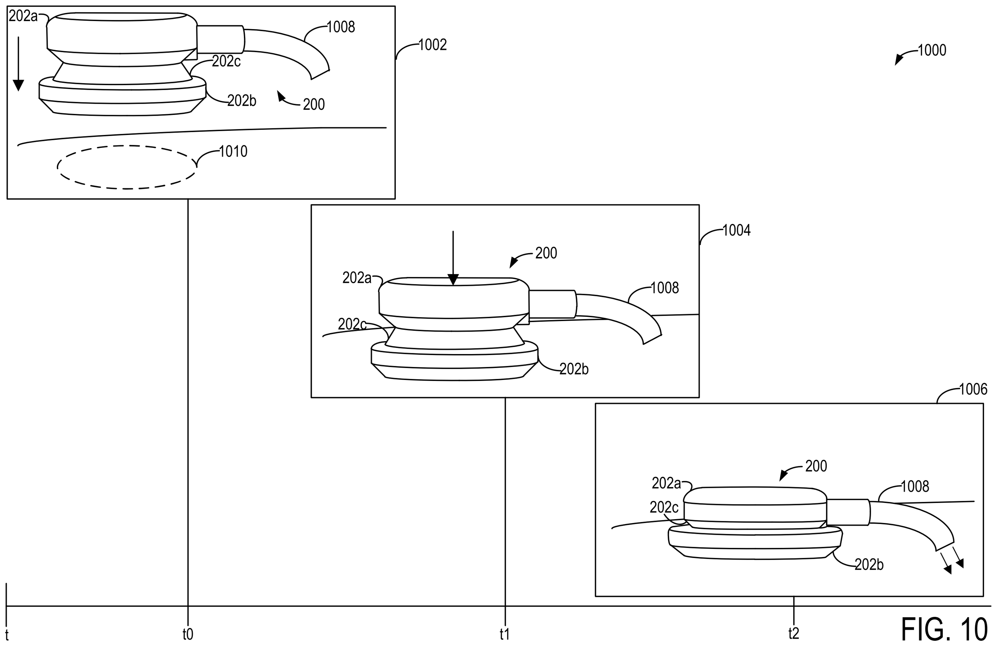

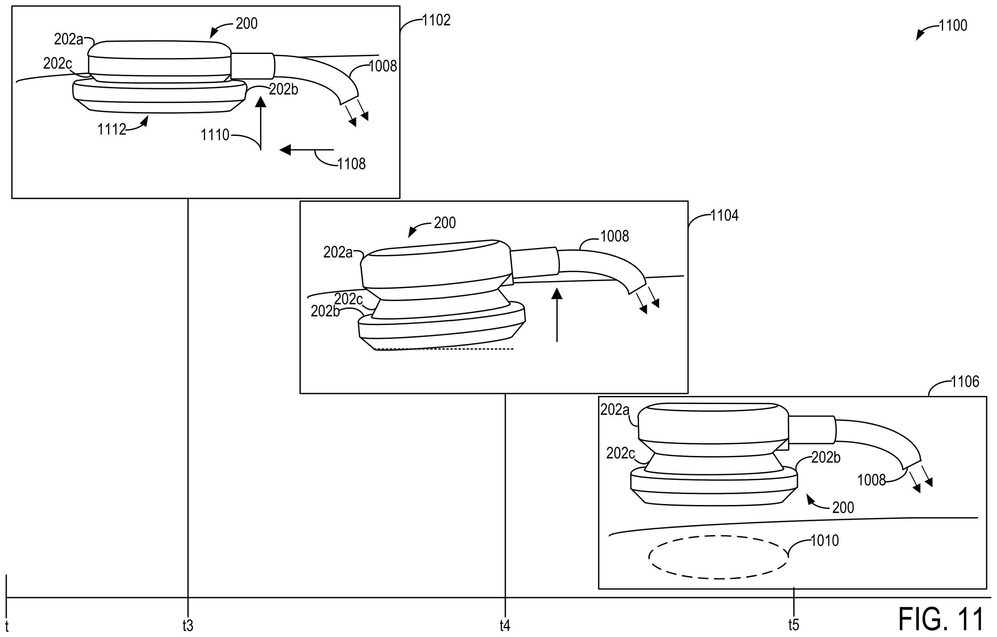

FIG. 6 shows an example of the suction electrode assembly in an actuated state. A first cross section of the suction electrode assembly depicted in FIG. 6 is illustrated in FIG. 7. A second cross section of the suction electrode assembly depicted in FIG. 6 is illustrated in FIG. 8. FIG. 9 shows an example method for obtaining ECG data with the suction electrode assembly. FIG. 10 depicts a timing diagram for achieving the actuated state of the suction electrode assembly, according to embodiments described herein. FIG. 11 depicts a timing diagram for returning to the non-actuated state of the suction electrode assembly according to embodiments described herein.

Referring to FIG. 1, an electrocardiogram (ECG) system 100 is shown, in accordance with an exemplary embodiment. ECG system 100 comprises a signal sensing unit, which may take different forms, including a standard 12-lead ECG. The 12-lead ECG, shown in FIG. 1, may include a set of electrodes 150, attached to a patient 140. Each electrode 150 may be the suction electrode assembly described herein with respect to FIGS. 2-8. Electrodes 150 are electrically coupled to a data acquisition module 118, thus enabling data acquisition module 118 to measure ECG waveform data by determining a difference in electrical potential between two or more electrodes of electrodes 150.

Data acquisition module 118 is communicatively coupled to ECG processing system 102, for processing, storing, and/or diagnosing, ECG data acquired by data acquisition module 118. ECG processing system 102 is further communicatively coupled to a display device 114, which is configured to display ECG data and/or diagnoses determined for the ECG data and may be part of a user interface, as well as user input device 116, which may enable a user to enter data into ECG processing system 102 or interact with data within non-transitory memory 106. Further, ECG system 100 may be communicatively coupled to one or more client devices (not shown), such as through a network connection, or through the Internet.

Data acquisition module 118 is configured to acquire time traces of electrical potentials between two or more of electrodes 150, which may include using an amplifier (not shown) to amplify ECG signals. In ECG system 100, electrodes 150 include ten electrodes, configured to measure a twelve lead ECG. In some embodiments, ECG system 100 may include 13 electrodes and may be configured to acquire fifteen lead ECG data. The methods and systems described herein apply to any multiple lead ECG system, including, but not limited to, a twelve lead ECG system.

Although the meaning of a twelve lead ECG will be well understood by a person having ordinary skill in the art, briefly, a twelve lead ECG provides a record of the electrical potential of the heart, as a function of time, measured along twelve distinct axes intersecting the heart. Therefore, a twelve lead ECG includes twelve distinct, time varying signals, wherein each of the twelve distinct time varying signals represents the electrical activity of the heart measured along a different axis. The electrodes 150 may include an electrically conductive gel that contacts the patient's skin and conducts to the electrode electrical signals that are present at the skin. The patient 140's heart produces an electrical signal that is referred to as an ECG waveform, and which may also be referred to herein as an ECG signal, an ECG lead signal, an ECG, or ECG data.

Specifically, electrodes 150 include four limb electrodes, including electrode 120 placed on a right arm of patient 140 (and therefore conventionally referred to as RA), electrode 122 placed on a right leg of patient 140 (and therefore conventionally referred to as RL), electrode 138 placed on a left arm of patient 140 (and therefore conventionally referred to as LA), and electrode 124 placed on a left leg of patient 140 (and therefore conventionally referred to as LL). The four limb electrodes may be used to measure the electrical potential of the heart along six distinct axes intersecting the heart, where each of the electrical potentials measured along a distinct axis is referred to as a lead. The six leads which the four limb electrodes are configured to measure are referred to as I (which generates the electrical potential difference between electrode 138 and electrode 120), II (which generates the electrical potential difference between electrode 124 and electrode 120), III (which generates the electrical potential difference between electrode 124 and electrode 138), aVR (which generates the electrical potential difference between electrode 120 and the average of electrodes 124 and 138), aVL (which generates the electrical potential difference between electrode 138 and the average of electrodes 120 and 124), and aVF (which generates the electrical potential difference between electrode 124 and the average of electrodes 120 and 138).

Further, electrodes 150 has six chest electrodes, including electrode 126 (conventionally referred to as V1), electrode 128 (conventionally referred to as V2), electrode 130 (conventionally referred to as V3), electrode 132 (conventionally referred to as V4), electrode 134 (conventionally referred to as V5), and electrode 136 (conventionally referred to as V6). The six chest electrodes, in conjunction with the limb electrodes, are configured to measure electrical potentials through six distinct axes intersecting the heart in a horizontal plane (that is, a plane perpendicular to the plane in which the six limb leads are measured). Specifically, the six leads which the six chest electrodes are configured to measure are referred to as V1 (which generates the potential difference between electrode 126 and the average of electrodes 120, 124, and 138), V2 (which generates the potential difference between electrode 128 and the average of electrodes 120, 124, and 138), V3 (which generates the potential difference between electrode 130 and the average of electrodes 120, 124, and 138), V4 (which generates the potential difference between electrode 132 and the average of electrodes 120, 124, and 138), V5 (which generates the potential difference between electrode 134 and the average of electrodes 120, 124, and 138), and V6 (which generates the potential difference between electrode 136 and the average of electrodes 120, 124, and 138).

ECG data acquired by data acquisition module 118 may be transmitted to ECG processing system 102 for storage, and processing (signal filtering, normalization, noise suppression, etc.). ECG processing system 102 may further be configured to automatically diagnose ECG data acquired by data acquisition module 118 by executing one or more operations of one or more of the methods herein disclosed. ECG processing system 102 includes a processor 104, and non-transitory memory 106, where processor 104 may read instructions from non-transitory memory 106 to execute one or more operations of one or more of the methods stored therein. ECG processing system 102 is further communicatively coupled to display device 114, and user input device 116, which may enable a user to see, and interact with, data within ECG processing system 102, respectively.

ECG processing system 102 includes the processor 104 configured to execute machine readable instructions stored in non-transitory memory 106. In one example, instructions for operating the suction electrode assembly to obtain ECG data based on signals received from pressure sensors as described in FIG. 9 may be stored in non-transitory memory. Processor 104 may be single core or multi-core, and the programs executed thereon may be configured for parallel or distributed processing. In some embodiments, the processor 104 may optionally include individual components that are distributed throughout two or more devices, which may be remotely located and/or configured for coordinated processing. In some embodiments, one or more aspects of the processor 104 may be virtualized and executed by remotely-accessible networked computing devices configured in a cloud computing configuration.

Non-transitory memory 106 further includes ECG data module 108, which includes ECG data acquired by one or more data acquisition modules or ECG systems. In some embodiments, ECG data module 108 includes data acquired by ECG system 100. In some embodiments, ECG data module 108 may store ECG data acquired through communicative coupling with one or more data sources other than ECG processing system 102. ECG data stored within ECG data module may be organized according to one or more organizational schemes, or configured into one or more data structures known in the art of data storage.

Non-transitory memory 106 further includes a vacuum system module 110, which includes instructions for operating a vacuum system 112 coupled to the ECG system 100 based on data received from the data acquisition module 118. For example, the plurality of electrodes 150 may include pressure sensors that generate and transmits signals to the data acquisition module 118 in response to a measured pressure being within one of a positive pressure threshold or a negative pressure threshold. Instructions stored in the vacuum system module 110 may be initiated based on pressure sensor data received from the data acquisition module 118.

In some embodiments, the non-transitory memory 106 may include components disposed at two or more devices, which may be remotely located and/or configured for coordinated processing. In some embodiments, one or more aspects of the non-transitory memory 106 may include remotely-accessible networked storage devices configured in a cloud computing configuration.

ECG system 100 further includes user input device 116. User input device 116 may comprise one or more of a touchscreen, a keyboard, a mouse, a trackpad, a motion sensing camera, or other device configured to enable a user to interact with and manipulate data within ECG processing system 102. As an example, user input device 116 may enable a user to make a selection of ECG data to diagnose. The user input device 116 may also enable the user to manually cease operation of the vacuum system 112.

Display device 114 may include one or more display devices utilizing virtually any type of technology. In some embodiments, display device 114 may comprise a computer monitor, and may display unprocessed and processed ECGs data. Display device 114 may be combined with processor 104, non-transitory memory 106, and/or user input device 116 in a shared enclosure, or may be peripheral display devices and may comprise a monitor, touchscreen, projector, or other display device known in the art, which may enable a user to view ECG data and/or interact with various data stored in non-transitory memory 106.

It should be understood that ECG system 100 shown in FIG. 1 is for illustration, not for limitation. Another appropriate ECG processing system, for example, an ICEG system, may include more, fewer, or different components.

FIG. 2 illustrates a suction electrode assembly 200 that may be integrated in an ECG system, such as the ECG system 100 of FIG. 1. The suction electrode assembly 200 is in a non-actuated state. The suction electrode assembly 200 includes a suction dome housing 202 fabricated from silicon and an electrode housing 204 wherein an electrode plate (not shown) is enclosed therein. A cylindrical portion of the electrode housing 204 extends from an interior of the suction dome housing 202 to an exterior of the suction dome housing. In this way, the cylindrical portion of the electrode housing 204 is coupled to a vacuum system that operates with an intermittent/variable flow rate or a constant flow rate and is electrically coupled to a data acquisition module, such as data acquisition module 118 of ECG system 100 of FIG. 1, via wiring (not shown) enclosed in tubing (not shown) coupled to the vacuum system.

The suction dome housing 202 includes a top portion 202a, a bottom portion 202b, and a conical middle portion 202c that is spaced between the top portion and the bottom portion. The top portion 202a comprises a first upper portion 206a that is generally cylindrical in shape with an upper base of the first upper portion having rounded edges and a first lower portion (not shown). A half-cylinder portion 208 extends from the first lower portion (not shown) on one end of the top portion 202a such that a base of the half-cylinder portion forms a continuous and smooth surface with the first upper portion 206a.

A diameter of the half-cylinder portion 208 is greater in size than a diameter of the cylindrical portion of the electrode housing 204. The half-cylinder portion 208 is coaxial with the cylindrical portion of the electrode housing 204. As such, one half of the half-cylinder portion 208 is located on one side of the suction dome housing 202 relative to the x-axis and another half of the half-cylinder portion 208 is located on another side of the suction dome housing 202 relative to the x-axis. The bottom portion 202b comprises a second upper portion 210a that is generally cylindrical in shape with an upper base of the second upper portion having rounded edges and a second lower portion 210b that is generally conical in shape.

FIG. 3 illustrates a first example 300 of a first cross section of the suction electrode assembly 200 depicted in FIG. 2. The first example 300 of the first cross section may share at least some of the structural and functional features with the suction electrode assembly 200 depicted in FIG. 2. Therefore, redundant description of these overlapping features may be omitted for concision. The first cross section extends in a direction of a XZ plane. As described in FIG. 2, the suction electrode assembly 200 includes the suction dome housing 202 and the electrode housing 204. The suction dome housing 202 includes the top portion 202a, the bottom portion 202b, and the conical middle portion 202c.

The electrode housing 204 is arranged with an interference fit with the suction dome housing 202 to introduce rigidity to the top portion 202a and to reduce air leaks from the top portion. More specifically, the top portion 202a is configured to withstand a force that depresses the top portion 202a and the conical middle portion 202c is configured to concentrically collapse in response to the top portion being depressed. The bottom portion 202b is configured to contact a surface (e.g., the skin of the patient).

The top portion includes the first upper portion 206a and a first lower portion 206b that is generally conical in shape. The first lower portion 206b deviates from the conical shape on one end of the suction electrode assembly where the half-cylinder portion 208 is positioned and extends from the first lower portion 206b. The first lower portion 206b transitions from the conical shape to a cylindrical shape at the location wherein the half-cylinder portion 208 is located.

The first upper portion 206a is contiguous with the first lower portion 206b. The first lower portion 206b is contiguous with an upper region of the conical middle portion 202c. The bottom portion 202b includes the second upper portion 210a and the second lower portion 210b. The second upper portion 210a is contiguous with the second lower portion 210b. The second upper portion 210a is contiguous with a lower region of the conical middle portion 202c. The second lower portion 210b is in contact with the surface (e.g., the skin of the patient).

The conical middle portion 202c has an axially varying wall thickness that couples the top portion 202a and the bottom portion 202b and functions as a membrane wherein air may flow from the suction dome housing 202 to the electrode housing 204 in response to the vacuum system operating. A wall thickness of the conical middle portion 202c is thinnest near where the conical middle portion transitions to the top portion 202a and the wall thickness of the middle portion is thickest near where the middle portion transitions to the bottom portion 202b. In particular, the wall thickness of the conical middle portion 202c is thinnest near the first lower portion 206b and the wall thickness of the conical middle portion is thickest near the second upper portion 210a.

The suction dome housing 202 includes an inner wall 306 that is configured such that a cross section of the suction dome housing 202 is circular in shape. The inner wall 306 of the suction dome housing 202 includes an upper region 306a and a lower region 306b. The cross section of the suction dome housing 202 is variable in diameter near the upper region 306a of the inner wall 306 and the cross section of the suction dome housing is constant in diameter near the lower region 306b of the inner wall. An end of the lower region 306b of the inner wall 306 defines the lip radius 308 of the suction dome housing 202. The lip radius 308 is sized to ensure that the patients experiences reduced irritation when the vacuum system is operating and the suction electrode assembly 200 is subjected to vacuum.

As described herein, the suction electrode assembly 200 includes the electrode housing 204 that is coupled to the vacuum system. The electrode housing 204 surrounds and positions an electrode plate 302, the electrode plate 302 being electrically coupled to the electrode housing 204 by a pin 304. Generally, the electrode housing 204 and the suction dome housing 202 are arranged such that the electrode plate 302 is generally positioned in a center of the suction dome housing. The pin 304 is offset from a center of the suction dome housing 202 and is positioned closer to the cylindrical portion of the electrode housing 204 than the center of the suction dome housing 202.

Signals generated by the electrode plate 302 may be electrically transmitted from the electrode plate to the pin 304, from the pin to the electrode housing 204, and from the electrode housing 204 to wires that electrically couple the electrode housing and a data acquisition module, such as the data acquisition module 118 of FIG. 1. The electrode housing 204 is electrically conductive and magnetically permeable to enable transmission of signals generated by the electrode plate 302 to the data acquisition module. In particular, the electrode housing 204 is fabricated from a plastic material that includes carbon. In this way, the plastic material has electrically conductivity and magnetic permeability that is desired for signal transmission.

FIG. 4 illustrates a second example 400 of the first cross section of the suction electrode assembly 200 depicted in FIG. 2. The second example 400 of the first cross section may share at least some of the structural and functional features with the suction electrode assembly 200 depicted in FIGS. 2 and 3. Therefore, redundant description of these overlapping features may be omitted for concision. The first cross section extends in the direction of the XY plane. The second example 400 of the first cross section of the suction electrode assembly 200 includes the suction dome housing 202 and the electrode housing 204.

The electrode housing 204 includes a first port 402 wherein one end of the first port 402 is coupled to the vacuum system and another end of the first port is fluidically coupled to a first cavity 404. The first port 402 includes a first tapered region 402a and a second tapered region 402b that are contiguous with each other and are arranged such that the first port 402 is continuous. The first tapered region 402a tapers more gradually compared to the second tapered region 402b.

The first cavity 404 is generally bowl shaped. The cross section of the first cavity 404 differs between a top, a middle, and a bottom of the first cavity. A cross section near the top of the first cavity 404 is circular and decreases in size due to tapering near the top of the first cavity. A cross section near the middle of the first cavity 404 is generally circular with half-circle cutouts positioned around a perimeter of the cross section. The cross section near the middle of the first cavity has a constant size.

A cross section near the bottom of the first cavity 404 decreases near a bottom of the first cavity due to tapering near the bottom of the first cavity. Since the pin 304 is positioned closer to the cylindrical portion of the electrode housing 204 and is not aligned with a center of the suction dome housing, the bottom of the first cavity 404 does not have a circular cross section. Instead, the cross section near bottom of the first cavity 404 is generally crescent-shaped due to the electrode housing 204 surrounding the pin 304.

The bottom of the first cavity 404 is fluidically coupled to a second port 408. The second port 408 is a tubular opening that allows air to flow from an interior 410 of the suction dome housing, or rather the interior of the conical middle portion 202c and the bottom portion 202b, to an interior (e.g., first cavity 404) of the electrode housing 204. From the interior of the electrode housing 204, the air may flow in a direction of the vacuum system through tubing coupled to the cylindrical portion of the electrode housing.

A connector 406 is positioned near a middle of the first cavity 404. In this way, the top of the first cavity is spaced apart from the bottom of the first cavity by the connector 406. A cross section of the connector 406 is generally circular in shape with half-circular cutouts positioned on a perimeter of the connector to enable the connector to be positioned near the middle of the first cavity 404. The connector 406 may be fabricated from silver chloride and coupled to the electrode housing 204 via an adhesive. The connector 406 is electrically coupled to the electrode housing 204.

Since the pin 304 is electrically coupled to the connector 406, it follows that the electrode plate 302 is electrically coupled to the connector 406 via the pin 304. Such a configuration of the various components of the suction electrode assembly 200 enables the electrode plate 302 to be electrically coupled to the electrode housing 204. In this way, signals generated by the electrode plate 302 may be transmitted to the data acquisition module (e.g., data acquisition module 118 of FIG. 1) via the electrode housing 204.

It may be understood that a configuration of the electrode housing may deviate from the example provided without departing from the scope of the present disclosure. For example, the shape and positioning of both the cavities and ports in the interior of the electrode housing may differ.

FIG. 5 illustrates an example of a second cross section 500 of the suction electrode assembly 200 depicted in FIG. 2. The second cross section 500 may share at least some of the structural and functional features with the suction electrode assembly 200 depicted in FIGS. 2-4. Therefore, redundant description of these overlapping features may be omitted for concision.

The second cross section extends in a direction of the YZ plane. The second cross section is perpendicular to a longitudinal axis of the cylindrical portion of the electrode housing 204. As such, the cylindrical portion of the electrode housing 204 is aligned with the pin 304 that electrically couples the electrode plate 302 to the electrode housing 204. The electrode plate 302 of the suction electrode assembly is positioned slightly below the top portion 202a and in a conical middle portion 202c of the suction dome housing 202 in the non-actuated state.

As described herein, the cross section near the bottom of the first cavity 404 is crescent-shaped, and therefore, the first cavity 404 surrounds the portion of the electrode housing 204 that encloses the pin 304 such that there is space on each side of the portion of the electrode housing enclosing the pin. The inner wall of the electrode housing 204 that is positioned near the middle of the first cavity 404 includes vertically positioned half-cylinders 502 that project into the first cavity. The vertically positioned half-cylinders 502 that project from the inner wall of the electrode housing 204 provide a surface for positioning the connector 406 in the middle portion of the first cavity 404. The surface created by the vertically positioned half-cylinders 502 ensures that the connector 406 is fixed within the middle of the first cavity 404 and coupled to the electrode housing 204 via an adhesive material. As such, movement of the connector 406 may be reduced.

FIG. 6 illustrates the suction electrode assembly 200 in an actuated state 600. The actuated state 600 of the suction electrode assembly 200 may share at least some of the structural and functional features with non-actuated state of the suction electrode assembly 200 depicted in FIGS. 2-5. Therefore, redundant description of these overlapping features may be omitted for concision.

The conical middle portion 202c of the suction dome housing 202 collapses concentrically and enters the actuated state 600 in response to negative pressure being generated when the top portion 202a is depressed and released. In the actuated state, the top portion 202a is positioned downward compared to the non-actuated state. The bottom portion 202b maintains a round shape and an electrode plate 302 contacts the surface in the actuated state 600.

Downward vertical movement of the conical middle portion 202c is hindered by the half-cylinder portion 208 of the suction dome housing 202 contacting the bottom portion 202b. As such, the half-cylinder portion 208 is positioned downward such that the half-cylinder portion is in contact with the bottom portion 202b at some locations along the half-cylinder portion and is not in contact with the bottom portion at other locations along the half-cylinder portion. Further, the cylindrical portion of the electrode housing 204 shifts downward such that the cylindrical portion of the electrode housing 204 is nearly touching but is not in contact with the bottom portion 202b of the suction dome housing 202.

FIG. 7 illustrates a first cross section 700 of the suction electrode assembly in the actuated state 600. The first cross section 700 of the actuated state 600 of the suction electrode assembly 200 may share at least some of the structural and functional features with the suction electrode assembly 200 depicted in FIGS. 2-6. Therefore, redundant description of these overlapping features may be omitted for concision.

The first cross section 700 extends in the direction of the XY plane. As described herein, the actuated state 600 of the suction electrode assembly 200 is shifted downward compared to the non-actuated due to the downward vertical movement of the conical middle portion 202c of the suction dome housing 202. More specifically, the conical middle portion 202c shifts downward in a vertical direction (such that the first lower portion 206b of the top portion 202a is positioned near the second upper portion 210a of the bottom portion 202b. The conical middle portion 202c may experience slight horizontal movement if an external force is applied. However, the slight horizontal movement does not affect an ability of the electrode plate to contact the surface (e.g., the skin of the patient).

Additionally, the half-cylinder portion 208 of the suction dome housing 202 encounters the second upper portion 210a of the bottom portion 202b, which hinders further vertical movement of the conical middle portion 202c as well as the top portion 202a. It follows that the inner wall 306 of the suction dome housing decreases in height, which in turn, decreases the volume of the interior 410 of the suction dome housing. As such, the electrode plate is positioned closer to the bottom portion 202b of the suction dome housing 202, and thus, the surface (e.g., the skin of the patient).

FIG. 8 illustrates a second cross section 800 of the suction electrode assembly in the actuated state 600. The second cross section 800 of the actuated state 600 of the suction electrode assembly 200 may share at least some of the structural and functional features with the suction electrode assembly 200 depicted in FIGS. 2-7. Therefore, redundant description of these overlapping features may be omitted for concision. The second cross section 800 extends in the direction of the YZ plane.

Compared to the non-actuated state of the suction electrode assembly, the position of the electrode plate 302 moves from being slightly below the top portion 202a to being positioned in a middle region of the bottom portion 202b of the suction dome housing for vacuum flow rates above a flow rate threshold. The vertical displacement of the conical middle portion 202c results in bending of the conical middle portion 202c. An extent of bending is greatest near a bottom of the conical middle portion 202c and a smallest extent of bending is near a top of the conical middle portion.

The conical middle portion 202c is bent in such a way that the bottom of the conical middle portion 202c is positioned higher than the top of the conical middle portion for vacuum flow rates above a flow rate threshold. The relative placement of the various components positioned in the interior of the electrode housing 204 remains the same in the actuated state 600 compared to the non-actuated state. As such, being exposed to vacuum does not alter the placement of the various components in the interior of the electrode housing 204. In scenarios wherein the flow rate of the vacuum system is below the flow rate threshold for the vacuum and the surface has flexibility (e.g., looser skin of a patient), the conical middle portion 202c may not collapse. Instead, the shape of the conical middle portion 202c may remain in the non-actuated state and the surface (e.g., the skin of the patient) is pulled towards the electrode plate despite the conical middle portion 202c not collapsing.

The various features of the suction electrode assembly 200 depicted in FIGS. 2-8 are designed to increase the holding force of the suction electrode housing when exposed to vacuum. For example, the wall thickness of the top portion 202a, the bottom portion 202b, and the conical middle portion 202c, heights, diameter sizes, surface finish, and hardness of each of the top portion 202, the bottom portion 202, and the conical middle portion, and lip radius are selected to increase holding force at difference operating conditions.

The different operating conditions may include dry surfaces, wet surface (e.g., due to sweat on a surface of the skin), non-uniform surfaces (e.g., due to hairy skin), soft tissue, hard tissue, and the like. In this way, the likelihood that the ECG data may be rendered non-diagnostic due to artifacts from improper contact between the electrode plate and skin from movement of the lead wires, movement of the patient during standard testing and stress testing, and the like may be reduced. In turn, this may reduce ECG cycle times and reduce times demanded for determining a diagnosis and/or monitoring a current diagnosis.

Further, the suction electrode assembly 200 operates in conjunction with the vacuum system to reduce leaks at the different operating conditions, which in turn may reduce power consumption of the vacuum pump during operation of the vacuum system. Additionally, the suction electrode assembly 200 may operate in conjunction with the vacuum system to increase the vacuum, and thus the holding force, in response to lead wires being pulled. In this way, leaks and detachment of the suction electrode assembly 200 may be reduced or prevented altogether.

FIG. 9 illustrates a method 900 for obtaining ECG data with an ECG system that integrates the suction electrode assembly. Method 900 is described with regard to the systems and components of FIGS. 1-8, though it should be appreciated that the method 900 may be implemented with other systems and components without departing from the scope of the present disclosure. Method 900 may be carried out according to instructions stored in non-transitory memory of a computing device, such as non-transitory memory 106 of FIG. 1.

At 902, the method 900 includes positioning a suction electrode assembly on a skin of a patient. The suction electrode assembly may be placed on the skin of a patient in a designated location according to various arrangements, including the standard 12-lead ECG, as depicted in FIG. 1. However, it may be understood that the suction electrode assembly may be placed on the skin of a patient in a designated location according to arrangements other than the standard 12-lead ECG. For example, the suction electrode assembly may be placed on the skin in a designated location according to a 15-lead ECG or 18-lead ECG.

At 904, the method 900 includes activating a vacuum system coupled to the suction electrode assembly in response to pressure within the suction electrode assembly being within one of a positive pressure threshold or a negative pressure threshold. The positive pressure threshold may be achieved by applying a force on a top portion of a suction dome housing. The suction dome housing is in a non-actuated state prior to applying the force to the top portion. A positive pressure is generated in response to the force being applied to the top portion of the suction dome housing. Force may be applied manually to the top portion of the suction dome housing by a user operating the ECG system.

Applying the force to the top portion of the suction dome housing causes air molecules confined in the interior of the suction dome housing to be compressed. Compression of the air molecules may generate a positive pressure within the suction dome housing. The negative pressure threshold may be achieved by releasing the force from the top portion of the suction dome housing. Releasing the force may cause generation of negative pressure due to volume expansion of the suction dome housing. The suction electrode assembly includes sensors that transmit a signal to the ECG system, and thus the vacuum system, to actuate a vacuum pump that pulls vacuum on the suction electrode assembly.

At 906, the method 900 includes removing the force from the top portion in response to the vacuum pump being activated or to activate the vacuum pump to achieve an actuated state and enable the suction dome housing to concentrically collapse. Removing the force may generate a negative pressure due to volume expansion of the suction dome housing. As such, the conical middle portion of the suction dome housing concentrically collapses in response to negative pressure being generated when the force is removed from the top portion of the suction dome housing and vacuum being generated by the vacuum pump.

Accordingly, a position of each of the top portion, the electrode housing, and the electrode plate changes in response to removing the force from the top portion to achieve the actuated state of the suction dome housing, and thus, the suction electrode assembly. More specifically, the position of each of the top portion, the electrode housing, and the electrode is shifted downward when the conical middle portion collapses to ensure the electrode plate is able to contact the skin of the patient.

For example, when the vacuum flow rates are above a flow rate threshold, the electrode plate is positioned slightly below the top portion and in a conical middle portion of the suction dome housing in the non-actuated state. When the position of the top portion and the electrode housing are moved downward, the position of the electrode plate moves from being slightly below the top portion to being positioned in a middle region of the bottom portion of the suction dome housing. When the vacuum flow rates are below the flow rate threshold, the position of the top portion may and the position of the conical middle portion may stay relatively the same (e.g., in the non-actuated state), such that the electrode plate is positioned above the bottom portion. Regardless of the vacuum flow rate, the skin of the patient may stretch in response to being exposed to vacuum, which in turn, enables contact between the skin of the patient and the electrode plate. However, vacuum flow rates below the flow rate threshold may demand more flexible skin (e.g., looser skin) compared to vacuum flow rates above the flow rate threshold to ensure that the electrode plate and the skin are in contact. In this way, the electrode plate may measure electrical potential at the skin of the patient, generate a signal based on the electrical potential, and transmits the signal to a data acquisition module, such as the data acquisition module 118 depicted in FIG. 1.

At 908, the method 900 includes collecting ECG data using the electrode enclosed within the suction dome housing. ECG data may comprise one or more lead signals/waveforms, indicating an electrical activity of a heart through time. In some embodiments, acquiring ECG data may comprise measuring a twelve lead ECG using ten electrodes enclosed within suction dome housings in electrical contact with a patient, as described in more detail above, with reference to FIG. 1. In some embodiments, acquiring ECG data may comprise measuring a fifteen lead ECG using thirteen electrodes enclosed within suction dome housings. In some embodiments, acquiring ECG data may comprise measuring a reduced lead ECG using less than ten electrodes, each electrode being positioned within a suction dome housing.

Acquired ECG data may be stored in memory for later processing. In some embodiments, the ECG data comprises 2D data of electrical potential through time, wherein electrical potential is plotted along the y-axis, and time is plotted along the x-axis. The ECG data acquired may be stored in one or more formats, including SCP-ECG, DICOM-ECG, HL7 aECG, and other storage formats known in the art of ECGs. Acquisition of ECG data may occur under various conditions, such as when the patient is resting, during exercise, or in an ambulance.

At 910, the method 900 includes removing negative pressure or generating a leak by applying force to a bottom portion of the suction dome housing to remove the electrode assembly from the skin. In one example, negative pressure may be removed from the suction dome housing in response to user input being received by the vacuum system. The ECG system, and thus the vacuum system, may receive user input that causes the vacuum pump and the vacuum system to cease operation.

In another example, negative pressure may be removed by generating the leak by applying force to a bottom portion of the suction dome housing. In particular, a force with both a horizontal and vertical component may be applied to a lip of the bottom portion to generate a leak. The suction dome housing of the suction electrode assembly returns to the non-actuated state in response to negative pressure being removed or a leak being generated due to the suction dome housing returning to atmospheric conditions. The method 900 then returns.

FIGS. 10 and 11 illustrate a first timing diagram 1000 and a second timing diagram 1100, respectively. The first timing diagram 1000 of FIG. 10 depicts the suction electrode assembly 200 being placed on a skin of a patient and entering the actuated state 600 of the suction electrode assembly. In contrast, the second timing diagram 1100 of FIG. 11 depicts the suction electrode assembly 200 being removed from the skin of the patient and returning to the non-actuated state of the suction electrode assembly. The suction electrode assembly may be integrated in an ECG system, such as the ECG system 100 described in FIG. 1.

For example, the suction electrode assembly 200 may be one electrode of a plurality of electrodes configured to measure electrical potential generated at a skin of a patient. Like the suction electrode assembly 200, each electrode is enclosed in an electrode housing coupled to a vacuum system and arranged with an interference fit with a suction dome housing that concentrically collapses to achieve an actuated state wherein the respective electrode is in contact with the skin of the patient.

In some examples, each electrode (e.g., suction electrode assembly 200) may include a sensor that measures a pressure for the electrode, or rather, the pressure within the suction dome housing. In other examples, a single pressure sensor may monitor pressure for all of the plurality of electrodes. Therefore, the vacuum system may be activated to compensate for vacuum pressure losses due to leaks that are present in at least one of the suction electrode assemblies. Accordingly, once the sensor measures a vacuum pressure that indicates a pressure threshold is satisfied, the vacuum pump, and thus the vacuum system may cease to operate.

The sensor transmits is communicatively coupled to the ECG system and thus, the vacuum system. In this way, the sensor may measure pressure, and in response to measuring pressure within one of a positive pressure threshold and a negative pressure threshold, a sensor signal may be generated and transmitted to the ECG system and from the ECG system to the vacuum system.

The ECG system includes processor 104, which is configured to execute machine readable instructions stored in a non-transitory memory 106 that cause the processors to activate the vacuum system in response to receiving a sensor signal that indicates pressure is within one of the positive pressure threshold or the negative pressure threshold, measure electrical potential data generated at a skin of a patient for each desired electrode and generate electrocardiograms based on the electrical potential data collected, and inactivate the vacuum system in response to user input. The electrical potential data generated at the skin of the patient may be measured for each electrode when each electrode is in the actuated state and the suction dome housing of each electrode returns to a non-actuated state in response to the vacuum system being inactivated.

Returning to FIG. 10, at a time t0, a first box 1002 depicts the suction electrode assembly 200 in a non-actuated state. In the first box 1002, the suction electrode assembly 200 is being placed on a designated area 1010 on the skin of the patient. The designated area 1010 is determined based on the configuration of the ECG system (e.g., 12-lead ECG, 10-lead ECG, etc.). The suction electrode assembly 200 is coupled to a vacuum system comprising a vacuum pump via tubing 1008. The tubing 1008 may also enclose electrical wiring that is electrically coupled to an electrode plate positioned within the suction electrode assembly 200 and a data acquisition module, such as data acquisition module 118 of FIG. 1. In this way, electrical potential data and pressure data may be transmitted to the data acquisition module.

At a time t1, a second box 1004 depicts force being applied to the suction electrode assembly 200. The suction electrode assembly 200 is in the non-actuated state in the second box 1004. Accordingly, the bottom portion 202b of the suction electrode assembly 200 is placed on the designated area 1010. The electrode plate of the suction electrode assembly 200 is not in contact with the designated area 1010 in the non-actuated state. To achieve the actuated state of the suction electrode assembly 200, a downward force is applied to a top portion 202a of the suction dome housing. The downward force may be achieved by a user manually depressing the top portion 202a with their hand.

At a time t2, a third box 1006 depicts the suction electrode assembly 200 in the actuated state. The force applied to the top portion 202a of the suction dome housing compresses air trapped beneath the suction dome housing. In turn, a positive pressure is generated which is measured by a pressure sensor of the suction electrode assembly 200. The pressure sensor includes thresholds for sensing both positive and negative pressures. Therefore, the pressure sensor generates a signal for the positive pressure and transmits the pressure signal to the ECG system and the vacuum system, which in turn, actuates the vacuum pump of the vacuum system to subject an interior of the suction electrode assembly 200 to vacuum.

Alternatively, the positive pressure generated by applying force to the top portion 202a may be insufficient for activating the vacuum pump. Instead, when the force is removed from the top portion 202a, a negative pressure may be generated due to volume expansion. Similarly, the pressure sensor generates a signal for the negative pressure and transmits the pressure signal and transmits the pressure signal to the ECG system and the vacuum system to actuate the pump to subject the interior of the suction electrode assembly 200 to vacuum.

As depicted, in the actuated state, the conical middle portion 202c concentrically collapses in response to the interior of the suction electrode assembly 200 being subjected to vacuum, causing the conical middle portion 202c and the top portion 202a to shift downward. The vertical displacement of the conical middle portion 202c and the top portion 202a causes the electrode plate to shift downward as well. Accordingly, due to being exposed to vacuum, the skin of the patient stretches toward the electrode plate such that there is adequate contact between the electrode plate and the skin of the patient to collect electrical potential measurements. However, as mentioned herein, when vacuum flow rates are below a certain threshold, the suction dome housing 202 may remain in the non-actuated state and contact between the skin of the patient and the electrode plate may occur without the conical middle portion 202c concentrically collapsing.

Returning to FIG. 11, the second timing diagram 1100 depict a sequence of timing events that occur a duration of time after the first timing diagram 1000. At a time t3, a fourth box 1102 depicts the suction electrode assembly 200 in the actuated state. The electrode enclosed within the suction electrode assembly 200 has collected the demanded ECG data. The suction electrode assembly 200 may return to the actuated state in response to negative pressure being removed from the suction electrode assembly 200 (e.g., no longer being subjected to vacuum).

The second timing diagram 1100 illustrates the negative pressure being removed when a leak is generated. Alternatively, user input entered by a user may result in the vacuum pump ceasing operation. In the fourth box 1102, a force with a horizontal component 1108 and a vertical component 1110 is applied to a lip 1112 of the suction dome housing. The lip 1112 is a portion of the bottom portion 202b (e.g., the portion of the second lower portion) that is in contact with the designated area 1010 of the skin of the patient.

At a time t4, the fifth box 1104 shows a leak being generated and negative pressure being removed from the suction electrode assembly 200. Since the negative pressure has been removed, and the suction electrode assembly 200 is no longer subjected to vacuum, the suction electrode assembly returns to the non-actuated state wherein the conical middle portion 202c is no longer concentrically collapsed in. As such, a height of the conical middle portion 202c returns to an original height of the conical middle portion, and thus, an overall height of the suction electrode assembly returns to an original overall height. At a time t5, a fifth box 1106 illustrates the suction electrode assembly in the non-actuated state removed from the designated area 1010 of the skin of the patient.

The technical effect of integrating a suction electrode assembly in an ECG system is that the amount of non-diagnostic ECG data collected may be reduced due to the suction electrode assembly being configured with a desired holding force between the electrode and the skin of the patient, which enables the suction electrode assembly to operate under various operating conditions. In this way, a number of motion artifacts and other artifacts included in the ECG data may be reduced.

The disclosure also provides support for a suction electrode assembly, comprising: an electrode housing that surrounds and positions an electrode plate, the electrode housing being coupled to a vacuum system, a suction dome housing configured to position the electrode housing and comprises a top portion, a bottom portion, and a conical middle portion with an axially varying wall thickness that couples the top portion and the bottom portion, and wherein the top portion is configured to withstand a force that depresses the top portion, the conical middle portion is configured to concentrically collapse in response to the top portion being depressed, and the bottom portion is configured to contact a surface. In a first example of the system, the vacuum system is a non-continuous suction system or intermittent suction system. In a second example of the system, optionally including the first example, the top portion comprises a first upper portion that is generally cylindrical in shape with an upper base of the first upper portion having rounded edges and a first lower portion that is generally conical in shape, the first upper portion being contiguous with the first lower portion. In a third example of the system, optionally including one or both of the first and second examples, a half-cylinder portion extends from the first lower portion on one side of the top portion such that a base of the half-cylinder portion forms a continuous and smooth surface with the first upper portion. In a fourth example of the system, optionally including one or more or each of the first through third examples, a cylindrical portion of the electrode housing extends from the continuous and smooth surface formed by the first lower portion and the first upper portion. In a fifth example of the system, optionally including one or more or each of the first through fourth examples, the bottom portion comprises a second upper portion that is generally cylindrical in shape with an upper base of the second upper portion having rounded edges and a second lower portion that is generally conical in shape. In a sixth example of the system, optionally including one or more or each of the first through fifth examples, the electrode housing is arranged with an interference fit with the suction dome housing to introduce rigidity to the top portion and reduce air leaks. In a seventh example of the system, optionally including one or more or each of the first through sixth examples, a wall thickness of the conical middle portion is thinnest near where the conical middle portion transitions to the top portion and the wall thickness of the conical middle portion is thickest near where the conical middle portion transitions to the bottom portion. In a eighth example of the system, optionally including one or more or each of the first through seventh examples, the conical middle portion of the suction dome housing collapses concentrically and enters an actuated state in response to negative pressure being generated when the top portion is depressed and released. In a ninth example of the system, optionally including one or more or each of the first through eighth examples, the bottom portion maintains a round shape and the electrode plate contacts the surface in the actuated state. In a tenth example of the system, optionally including one or more or each of the first through ninth examples, the surface is skin of a patient.

The disclosure also provides support for a method, comprising: positioning a suction electrode assembly on a skin of a patient, activating a vacuum pump coupled to the suction electrode assembly in response to receiving a sensor signal that indicates pressure inside the suction electrode assembly is within one of a positive pressure threshold or a negative pressure threshold, the positive pressure threshold being achieved by applying force on a top portion of a suction dome housing that encloses the suction electrode assembly to generate positive pressure and the negative pressure threshold being achieved by removing force from the top portion of the suction dome housing, removing force from the top portion in response to the vacuum pump being activated to concentrically collapse a conical middle portion of the suction dome housing to achieve an actuated state and to enable contact between the suction electrode assembly and the skin of the patient, collecting ECG data using the suction electrode assembly enclosed within the suction dome housing, and removing negative pressure by deactivating the vacuum pump or generating a leak by applying force to a bottom portion of the suction dome housing to remove the suction electrode assembly from the skin. In a first example of the method, the suction dome housing is in non-actuated state prior to applying force to the top portion. In a second example of the method, optionally including the first example, an electrode plate of the suction electrode assembly is positioned slightly below the top portion and in a middle portion of the suction dome housing in the non-actuated state for vacuum flow rates above a flow rate threshold. In a third example of the method, optionally including one or both of the first and second examples, a position of the electrode plate changes in response to removing force from the top portion to achieve the actuated state of the suction dome housing. In a fourth example of the method, optionally including one or more or each of the first through third examples, the position of the electrode plate moves from being slightly below the top portion to being positioned in a middle region of the bottom portion of the suction dome housing for vacuum flow rates above the flow rate threshold. In a fifth example of the method, optionally including one or more or each of the first through fourth examples, the suction dome housing returns to a non-actuated state in response to negative pressure being removed or the leak being generated.

The disclosure also provides support for a system, comprising: a plurality of electrodes configured to measure electrical potential generated at a skin of a patient, each electrode being enclosed in an electrode housing coupled to a vacuum system and arranged with an interference fit with a suction dome housing that concentrically collapses to achieve an actuated state wherein a respective electrode is in contact with the skin of the patient, an electrode monitor configured to generate an electrocardiogram (ECG) signal from the electrical potential measured by the plurality of electrodes, an interface for communicating with a user, at least one processor configured to execute stored instructions to: activate the vacuum system in response to a sensor signal that indicates pressure is within one of a positive pressure threshold or a negative pressure threshold, measure electrical potential data generated at the skin of the patient for each desired electrode and generate electrocardiograms based on the electrical potential data collected, and inactivate the vacuum system in response to user input. In a first example of the system, measuring electrical potential data generated at the skin of the patient for each electrode occurs when each electrode is in the actuated state. In a second example of the system, optionally including the first example, the suction dome housing returns to a non-actuated state in response to the vacuum system being inactivated.

FIGS. 1-8 show example configurations with relative positioning of the various components. If shown directly contacting each other, or directly coupled, then such elements may be referred to as directly contacting or directly coupled, respectively, at least in one example. Similarly, elements shown contiguous or adjacent to one another may be contiguous or adjacent to each other, respectively, at least in one example. As an example, components laying in face-sharing contact with each other may be referred to as in face-sharing contact. As another example, elements positioned apart from each other with only a space there-between and no other components may be referred to as such, in at least one example. As yet another example, elements shown above/below one another, at opposite sides to one another, or to the left/right of one another may be referred to as such, relative to one another. Further, as shown in the figures, a topmost element or point of element may be referred to as a "top" of the component and a bottommost element or point of the element may be referred to as a "bottom" of the component, in at least one example. As used herein, top/bottom, upper/lower, above/below, may be relative to a vertical axis of the figures and used to describe positioning of elements of the figures relative to one another. As such, elements shown above other elements are positioned vertically above the other elements, in one example. As yet another example, shapes of the elements depicted within the figures may be referred to as having those shapes (e.g., such as being circular, straight, planar, curved, rounded, chamfered, angled, or the like). Further, elements shown intersecting one another may be referred to as intersecting elements or intersecting one another, in at least one example. Further still, an element shown within another element or shown outside of another element may be referred as such, in one example.

As used herein, an element or step recited in the singular and preceded with the word "a" or "an" should be understood as not excluding plural of said elements or steps, unless such exclusion is explicitly stated. Furthermore, references to "one embodiment" of the present invention are not intended to be interpreted as excluding the existence of additional embodiments that also incorporate the recited features. Moreover, unless explicitly stated to the contrary, embodiments "comprising," "including," or "having" an element or a plurality of elements having a particular property may include additional such elements not having that property. The terms "including" and "in which" are used as the plain-language equivalents of the respective terms "comprising" and "wherein." Moreover, the terms "first," "second," and "third," etc. are used merely as labels, and are not intended to impose numerical requirements or a particular positional order on their objects.

This written description uses examples to disclose the invention, including the best mode, and also to enable a person of ordinary skill in the relevant art to practice the invention, including making and using any devices or systems and performing any incorporated methods. The patentable scope of the invention is defined by the claims, and may include other examples that occur to those of ordinary skill in the art. Such other examples are intended to be within the scope of the claims if they have structural elements that do not differ from the literal language of the claims, or if they include equivalent structural elements with insubstantial differences from the literal languages of the claims.

## Claims

1. A suction electrode assembly, comprising:
an electrode housing (204) that surrounds and positions an electrode plate (302), the electrode housing being coupled to a vacuum system (112);
a suction dome housing (202) configured to position the electrode housing and comprises a top portion (202a), a bottom portion (202b), and a conical middle portion (202c) with an axially varying wall thickness that couples the top portion and the bottom portion; and
wherein the top portion is configured to withstand a force that depresses the top portion, the conical middle portion is configured to concentrically collapse in response to the top portion being depressed, and the bottom portion is configured to contact a surface.

2. The suction electrode assembly of claim 1, wherein the vacuum system is a non-continuous suction system or intermittent suction system.

3. The suction electrode assembly of claim 1, wherein the top portion comprises a first upper portion (206a) that is generally cylindrical in shape with an upper base of the first upper portion having rounded edges and a first lower portion (206b) that is generally conical in shape, the first upper portion being contiguous with the first lower portion.

4. The suction electrode assembly of claim 3, wherein a half-cylinder portion (208) extends from the first lower portion (206b) on one side of the top portion such that a base of the half-cylinder portion forms a continuous and smooth surface with the first upper portion (206a).

5. The suction electrode assembly of claim 4, wherein a cylindrical portion of the electrode housing (204) extends from the continuous and smooth surface formed by the first lower portion (206b) and the first upper portion (206a).

6. The suction electrode assembly claim 1, wherein the bottom portion (202b) comprises a second upper portion (210a) that is generally cylindrical in shape with an upper base of the second upper portion having rounded edges and a second lower portion (210b) that is generally conical in shape.

7. The suction electrode assembly claim 1, wherein the electrode housing (204) is arranged with an interference fit with the suction dome housing (202) to introduce rigidity to the top portion (202a) and reduce air leaks.

8. The suction electrode assembly claim 1, wherein a wall thickness of the conical middle portion (202c) is thinnest near where the conical middle portion transitions to the top portion (202a) and the wall thickness of the conical middle portion is thickest near where the conical middle portion transitions to the bottom portion (202b).

9. The suction electrode assembly of claim 8, wherein the conical middle portion (202x) of the suction dome housing (202) collapses concentrically and enters an actuated state in response to negative pressure being generated when the top portion (202a) is depressed and released and the bottom portion (202b) maintains a round shape and the electrode plate (302) contacts the surface in the actuated state.

10. The suction electrode assembly of claim 1, wherein the surface is skin of a patient.

11. A method, comprising:
positioning (902) a suction electrode assembly on a skin of a patient;
activating (904) a vacuum pump coupled to the suction electrode assembly in response to receiving a sensor signal that indicates pressure inside the suction electrode assembly is within one of a positive pressure threshold or a negative pressure threshold, the positive pressure threshold being achieved by applying force on a top portion of a suction dome housing that encloses the suction electrode assembly to generate positive pressure and the negative pressure threshold being achieved by removing force from the top portion of the suction dome housing;
removing (906) force from the top portion in response to the vacuum pump being activated to concentrically collapse a conical middle portion of the suction dome housing to achieve an actuated state and to enable contact between the suction electrode assembly and the skin of the patient;
collecting (908) ECG data using the suction electrode assembly enclosed within the suction dome housing; and
removing (910) negative pressure by deactivating the vacuum pump or generating a leak by applying force to a bottom portion of the suction dome housing to remove the suction electrode assembly from the skin.

12. The method of claim 11, wherein the suction dome housing is in non-actuated state prior to applying force to the top portion and wherein an electrode plate of the suction electrode assembly is positioned slightly below the top portion and in a middle portion of the suction dome housing in the non-actuated state for vacuum flow rates above a flow rate threshold.

13. The method of claim 12, wherein a position of the electrode plate changes in response to removing force from the top portion to achieve the actuated state of the suction dome housing.

14. The method of claim 13, wherein the position of the electrode plate moves from being slightly below the top portion to being positioned in a middle region of the bottom portion of the suction dome housing for vacuum flow rates above the flow rate threshold.

15. The method of claim 11, wherein the suction dome housing returns to a non-actuated state in response to negative pressure being removed or the leak being generated.
